# EUROPEAN PATENT SPECIFICATION

(11) **EP 0 621 900 B1**
(45) Date of publication and mention of the grant of the patent: **26.06.1996**
(21) Application number: 93902955.9
(22) Date of filing: 06.01.1993
(51) Int. Cl.: C12P 7/40, C12N 15/53

(54) **OXIDATION OF GLYCOLIC ACID TO GLYOXYLIC ACID USING A MICROBIAL CELL TRANSFORMANT AS CATALYST**
Oxydation von Glykolsäure zur Herstellung von Glyoxylsäure unter Verwendung von transformierten mikrobiellen Zellen als Katalysator.
OXYDATION D'ACIDE GLYCOLIQUE EN ACIDE GLYCOXYLIQUE A L'AIDE D'UNE CELLULE TRANSFORMEE MICROBIENNE UTILISEE COMME CATALYSEUR

(30) Priority: 06.01.1992 US 817165
(43) Date of publication of application: 02.11.1994
(73) Proprietor: E.I. DU PONT DE NEMOURS AND COMPANY, Wilmington Delaware 19898 (US)
(72) Inventor: ANTON, David, Leroy, Wilmington, DE 19803 (US); DiCOSIMO, Robert, Wilmington, DE 19808 (US)
(74) Representative: Woodcraft, David Charles
(86) International application number: US9300077
(87) International publication number: WO9314214

(56) References cited:
- WO-A-91/05868
- CHEMICAL ABSTRACTS, vol. 112, no. 13, 26 March 1990, Columbus, OH (US); A. TANAKA et al., p. 239, no. 113606b
- CHEMICAL ABSTRACTS, vol. 109, no. 7, 15 August 1988, Columbus, OH (US); M. TAKAHASHI, p. 200, no. 49626s
- BIOCHEMISTRY, vol. 30, no. 18, 1991, Easton, PA (US); P. MACHEROUX et al., pp. 4612-4619

## Description

### BACKGROUND OF THE INVENTION

### 1. Field of the Invention:

This invention relates to an improved process for the production of glyoxylic acid by the enzyme catalyzed oxidation of glycolic acid. More specifically, the present invention relates to the use of whole cells of a genetically-engineered microbial transformant, which expresses the enzyme glycolate oxidase [(S)-2-hydroxy-acid oxidase, EC 1.1.3.15], and, optionally, catalase (EC 1.11.1.6).

### 2. Description of the Related Art:

Glycolate oxidase, an enzyme commonly found in leafy green plants and mammalian cells, catalyzes the oxidation of glycolic acid to glyoxylic acid, with the concomitant production of hydrogen peroxide. N. E. Tolbert et. al., J. Biol. Chem., Vol. 181, 905-914 (1949) first reported an enzyme, extracted from tobacco leaves, which catalyzed the oxidation of glycolic acid to formic acid and CO₂ via the intermediate formation of glyoxylic acid. The addition of certain compounds, such as ethylenediamine, limited the further oxidation of the intermediate glyoxylic acid. The oxidations were carried out at a pH of about 8, typically using glycolic acid concentrations of about 3-40 mM (millimolar). The optimum pH for the glycolate oxidation was reported to be 8.9. Oxalic acid (100 mM) was reported to inhibit the catalytic action of the glycolate oxidase. Similarly, K. E. Richardson and N. E. Tolbert, J. Biol. Chem., vol. 236, 1280-1284 (1961) showed that buffers containing tris(hydroxymethyl)aminomethane inhibited the formation of oxalic acid in the glycolate oxidase catalyzed oxidation of glycolic acid. C. O. Clagett, N. E. Tolbert and R. H. Burris, J. Biol. Chem., Vol. 178, 977-987 (1949) reported that the optimum pH for the glycolate oxidase catalyzed oxidation of glycolic add with oxygen was about 7.8 - 8.6, and the optimum temperature was 35-40°C.

I. Zelitch and S. Ochoa, J. Biol. Chem., Vol. 201, 707-718 (1953), and J. C. Robinson et. al., J. Biol. Chem., Vol. 237, 2001-2009 (1962), reported that the formation of formic acid and CO₂ in the spinach glycolate oxidase-catalyzed oxidation of glycolic acid resulted from the nonenzymatic reaction of H₂O₂ with glyoxylic acid. They observed that addition of catalase, an enzyme that catalyzes the decomposition of H₂O₂, greatly improved the yields of glyoxylic acid by suppressing the formation of formic acid and CO₂. The addition of flavin mononudeotide (FMN) was also found to greatly increase the stability of the glycolate oxidase.

N. A. Frigerio and H. A. Harbury, J. Biol. Chem., Vol. 231, 135-157 (1958) have reported on the preparation and properties of glycolic acid oxidase isolated from spinach. The purified enzyme was found to be very unstable in solution; this instability was ascribed to the relatively weak binding of flavin mononucleotide (FMN) to the enzyme active site, and to the dissociation of enzymatically active tetramers and/or octamers of the enzyme to enzymatically-inactive monomers and dimers, which irreversibly aggregate and precipitate. The addition of flavin mononucleotide (FMN) to solutions of the enzyme greatly increased its stability, and high protein concentrations or high ionic strength maintained the enzyme as octamers or tetramers.

There are numerous other references to the oxidation of glycolic acid catalyzed by glycolic acid oxidase, for example:
- Isolation of the enzyme (usually includes an assay method):
   I. Zelitch in Methods of Enzymology, Vol. 1, Academic Press, New York, 1955, p. 528-532, from spinach and tobacco leaves.
   M. Nishimura et al., Arch. Biochem. Biophys., Vol. 222, 397-402 (1983), from pumpkin cotyledons.
   H. Asker and D. Davies, Biochim. Biophys. Acta, Vol. 761, 103-108 (1983), from rat liver.
   M. J. Emes and K. H. Erismann, Int. J. Biochem., Vol. 16, 1373-1378 (1984), from Lemna Minor L.
- Structure of the enzyme:
   E. Cederlund et al., Eur. J. Biochem., Vol. 173, 523-530 (1988).
   Y. Lindquist and C. Branden, J. Biol. Chem. Vol. 264, 3624-3628, (1989).

### SUMMARY OF THE INVENTION

This invention relates to a process for the production of glyoxylic acid (OCHCOOH), where glycolic acid (HOCH₂COOH) (200 to about 2500 mM) and oxygen are reacted in an aqueous solution (pH 7 to 10) in the presence of whole cells of a genetically-engineered microbial transformant, which expresses the enzyme glycolate oxidase [(S)-2-hydroxy-acid oxidase, EC 1.1.3.15], and, optionally, catalase (EC 1.11.1.6). Under optimum conditions, very high yields of glyoxylic acid are obtained at high conversion of glycolic add, and the genetically-engineered microbial transformant can be recovered and reused.

The subject-matter of the invention is defined in claims 1-18.

### DETAILED DESCRIPTION OF THE INVENTION

This invention describes the use of whole cells of a microbial transformant (e.g., *Aspergillus nidulans, Pichia pastoris, Hansenulla polymorpha* and *Escherichia coli*) which co-expresses glycolate oxidase and catalase for the manufacture of glyoxylic acid from glycolic acid (hydroxyacetic acid). Although the enzyme-catalyzed reaction of glycolic acid with oxygen has been known for many years, high selectivities (> 99%) to glyoxylic acid have not been previously obtained, nor has the oxidation of glycolic acid been performed at concentrations of 0,20 M to 2.5 M. A previous, commonly assigned, application, U.S.S.N. 07/422,011, filed October 16,1989, "Production of Glyoxylic Acid from Glycolic Acid", (US-A-5 219 745 and US-A-5 221 621) described a process for the enzymatic conversion of glycolic acid to glyoxylic acid in the presence of oxygen, an amine buffer, and the soluble enzymes glycolate oxidase and catalase. This process demonstrated the unexpected synergistic effect of using both catalase (to destroy by-product hydrogen peroxide) and an amine buffer capable of forming a chemical adduct with the glyoxylic acid produced (limiting its further oxidation). Neither the separate addition of catalase or an amine buffer were found to produce the high selectivity observed when both were present, and the almost quantitative yields of glyoxylic acid obtained were more than expected from a simple additive effect of using catalase or amine buffer alone. The instant invention is viewed as an improvement to the above process in that the present invention uses a whole microbial cell as a catalyst, in place of the soluble enzymes.

The previously-reported use of soluble enzymes as catalysts poses several problems: catalyst recovery for reuse is not easily performed, catalyst stability is not as good as can be obtained with immobilized enzyme or whole cell microbial catalysts, and soluble enzymes are not stable to the sparging of the reaction mixture with oxygen (required to increase the rate of oxygen dissolution and, thus, reaction rate). Several transformants of *Aspergillus nidulans, Pichia pastoris, Hansenula polymorpha* and *Escherichia coli* have now been constructed, using genetic engineering techniques commonly known to those skilled in the art, which express the glycolate oxidase from spinach as well as an endogenous catalase. Several advantages are offered by the use of these whole cell catalysts in the previously described process: 1) the whole cell catalysts are easily recovered from the reaction mixture at the conclusion of the reaction for reuse, whereas the soluble enzyme is only recovered with great difficulty and loss of activity, 2) the whole cell catalysts are more stable than the soluble enzyme, both for the number of catalyst turnovers obtained versus the soluble enzyme, as well as for recovered enzyme activity at the conclusion of a reaction, and 3), most importantly, the whole cell catalyst are stable to reaction conditions where oxygen is sparged into the reaction mixture to increase the rate of oxygen dissolution and reaction rate, where under similar reaction conditions the soluble glycolate oxidase is rapidly denatured.

The *Aspergillus nidulans* transformants were prepared by first cloning the spinach gene which codes for glycolate oxidase and then introducing this gene into a strain of *Aspergillus nidulans* which already produced acceptable levels of the endogenous catalase. A genetically-engineered microbial transformant *Aspergillus nidulans* T17, harboring multiple copies of the spinach glycolate oxidase-encoding DNA under expression control of the *A. nidulans* alcA promoter, and multiple copies of the *A. nidulans* alkR gene, the product of which regulates function of the alcA promoter, was deposited under the terms of the Budapest Treaty with the Northern Regional Reasearch Center, Peoria Illinois, U.S.A. on September 24, 1992, under NRRL No.21000 The resulting transformants were cultured in various media (minimal or SYG rich media) in shaker flasks or fermenters, and additionally, different agents such as oleic acid (OL), hydroxyacetic acid (HA), or corn steep liquor (CSL) were added to the media to increase levels of expression of glycolate oxidase and/or catalase. The different transformants were then screened by assaying the *Aspergillus nidulans* whole cells (untreated) for catalase and glycolate oxidase activity, and by running reactions with the cells as catalysts for the oxidation of glycolic acid to glyoxylic acid. When used as catalysts for the oxidation of glycolic acid to glyoxylic acid, the whole cells were not pre-treated or permeabilized to increase accessibility of the reaction mixture to the enzymes in the interior of the cells; some permeabilization of the cells may take place, either from exposure to the reaction mixture or any of its components, or by freezing and thawing, which was used to store the whole cell catalysts until needed.

Many of the deficiencies of the soluble enzymes were eliminated by employing whole cells of *A. nidulans* as catalyst. Recovery and reuse of the whole-cell catalyst was easily performed by centrifugation or by filtering the catalyst away from the reaction mixture and recycling it to fresh reaction mixture; in this manner, turnover numbers for glycolate oxidase of as high as 10⁶ have been obtained. The ability to bubble oxygen through the reaction mixture without denaturing the enzyme catalyst (as is observed when using the soluble enzyme) resulted in increases in the reaction rate of at least ten-fold over reactions where the reaction mixture is not bubbled, and this increase in rate significantly reduces the cost of manufacture for this process.

Several additional microbial transformants which express glycolate oxidase activity as well as endogenous catalase activity have been prepared, and their use as a microbial catalyst in the present invention demonstrated. A second microbial cell catalyst which has been utilized in the present invention is a transformant of *Hansenula polymorpha* (a methylotrophic yeast). Several transformants of *H. polymorpha* having sufficient glycolate oxidase activity have been prepared by inserting the DNA for glycolate oxidase into an expression vector under the control of the formate dehydrogenase (FMD) promoter. *H. polymorpha* was transformed with this vector and a strain producing high levels of glycolate oxidase was selected and designated *H. polymorpha* GO1.

*H*. *polymorpha* cell catalysts were typically prepared by first growing an inoculum of an *H*. *polymorpha* transformant in 500 ml of YPD (Difco), pH 4.4. This culture was then inoculated into a fermenter containing 10 L of Yeast Nitrogen Base (YNB, Difco) without amino acids (14 g), ammonium sulfate (50 g) and methanol (100 g), at pH 5.0. The fermenter was operated for 42.5 h at 37°C, an agitation rate of 400 rpm, constant pH of 5.0, 40% dissolved oxygen (controlled), and 14 psig of air. At the conclusion of the fermentation, 1.0 kg of glycerol was added and the cells harvested by centrifugation, frozen in liquid nitrogen, and stored at -80°C.

A third microbial cell catalyst which has been utilized in the present invention is a transformant of *Pichia pastoris* (a methylotrophic yeast) which expresses the glycolate oxidase enzyme from spinach, as well as an endogenous catalase. Several transformants of *P. pastoris* having sufficient glycolate oxidase activity have been prepared by inserting a DNA fragment containing the spinach glycolate oxidase gene into a *P. pastoris* expression vector (pHIL-D4) such as to be under control of the methanol inducible alcohol oxidase I promoter, generating the plasmid pMP1. *P. pastoris* strain GTS115 (NRRL Y-15851) was transformed by plasmid pMP1 and a selection was done as to allow integration of the linearized plasmid pMP1 into the chromosomal alcohol oxidase I locus and replacement of alcohol oxidase gene with glycolate oxidase gene. A pool of such transformants were next selected for maximal number of integrated copies of the expression cassette. A high copy number transformant designated *P. pastoris* strain GS115-MSP10 was isolated and deposited in the NRRL, Peoria, Illinois (NRRL Y-21001, deposited September 24, 1992).

*P. pastoris* cells were typically prepared by growing an inoculum in 100 ml of YNB containing 1% glycerol. After 48 h growth at 30°C, the cells were transferred into a fermenter containing 10 L of media composed of yeast nitrogen base(YNB) without amino acids (134 g), glycerol (100 g), and biotin (20 mg). The fermentation was operated at pH 5.0 (controlled with NH₄OH), 30°C, agitation rate of 200 rpm, aeration of 5 slpm, 5 psig of air, and dissolved oxygen maintained at no lower than 50% saturation. When glycerol was depleted, the cells were induced to express glycolate oxidase by growth in the same media except that methanol (50 g) was substituted for glycerol. Glycolate oxidase activity during induction was followed by enzyme assay. After 24 h of induction the cells were harvested following treatment with glycerol (1 kg). Following harvest the cells were frozen in liquid nitrogen and stored at -80°C.

Unlike *A. nidulans*, *H. polymorpha* and *P. pastoris* cell transformants required permeabilization prior to use as catalyst for the oxidation of glycolic acid to glyoxylic acid. A variety of known methods of permeabilization were useful for preparing cells with sufficient glycolate oxidase activity (see Felix, H. Anal. Biochemistry, Vol. 120, 211-234, (1982)). Typically, a suspension of 10 wt % wet cells in 0.1 % (v/v) "TRITON®" X-100/20 mM phosphate buffer (pH 7.0) was mixed for 15 minutes, then frozen in liquid nitrogen, thawed, and washed with 20 mM phosphate/0.1 mM FMN buffer (pH 7.0). A second method of permeabilization was performed by mixing a suspension of 10 wt % wet cells in 0.1 % (w/v) benzalkonium chloride (Sigma)/20 mM phosphate buffer (pH 7.0) for 60 minutes, then washing the permeabilized cells with 20 mM phosphate/0.1 mM FMN buffer (pH 7.0).

A fourth microbial cell catalyst which has been utilized in the present invention is a transformant of *Escherichia coli* (a bacteria) which expresses the glycolate oxidase enzyme from spinach, as well as an endogenous catalase. Such an *E. coli* transformant was prepared as described in Macheroux et. al., Biochem. Biophys, Acta, Vol. 1132, 11-16 (1992).

The glycolate oxidase (added as *Aspergillus nidulans, Pichia pastoris, Hansenula polymorpha* or *Escherichia coli* whole cells) used in the reaction should be present in an effective concentration, usually a concentration of 0.01 to about 100 IU/mL, preferably about 0.1 to about 10 IU/mL. An IU (International Unit) is defined as the amount of enzyme that will catalyze the transformation of one micromole of substrate per minute. A procedure for the assay of this enzyme is found in I. Zelitch and S. Ochoa, J. Biol. Chem., Vol. 201, 707-718 (1953). This method is also used to assay the activity of recovered or recycled glycolate oxidase.

The pH of the reaction solution should be between 7 and 10, preferably between 8.0 and 9.5 The pH can be maintained by a buffer, since enzyme activity varies with pH. The pH of the reaction decreases slightly as the reaction proceeds, so it is often useful to start the reaction near the high end of the maximum enzyme activity pH range, about 9.0 - 9.5, and allow it to drop during the reaction. As has been previously described in U.S.S.N. 07/422,011, filed October 16, 1989, an amine buffer capable of complexing the glyoxylic acid (by forming an amine which is more stable to chemical or enzymatic oxidation) is employed along with catalase to maximize product selectivity. Ethylenediamine, or less preferably, tris(hydroxymethyl)aminomethane (hereinafter TRIS), piperazine, or glycylglycine improved the yield of glyoxylic acid. These amines are used in a molar ratio of amine/glycolic acid (starting amount) of 1.0 to 3.0, preferably 1.0 to 1.33. Within this range, the exact value may be adjusted to obtain the desired pH. With very basic amines used at high amine to glycolic acid ratios, it may be necessary to adjust the pH, as by adding acid, for example hydrochloric or sulfuric acids. With less basic amines such as TRIS, it may be necessary to add a base to maintain the desired pH.

The concentration of accessible catalase (added as *Aspergillus nidulans*, *Pichia pastoris, Hansenula polymorpha* or *Escherichia coli* whole cells) should be 50 to 100,000 IU/mL of reaction mixture, preferably 350 to 14,000 IU/mL. It is preferred that both the glycolate oxidase and catalase enzymes be present within the same microbial cell (in this case, a transformant of *A. nidulans*, *P. pastoris, H. polymorpha* or *E. coli),* but an additional source of microbial catalase (for example, but not by way of limitation, *Saccharomyces cerevisiae* or the like) may be added to supplement the catalase present. Additionally, the catalase and glycolate oxidase concentrations should be adjusted within the above ranges so that the ratio (measured in IU for each) of catalase:glycolate oxidase is at least about 250:1. Flavin mononucleotide (FMN) is an optional added ingredient, used at a concentration of 0.0 to 2.0 mM, preferably 0.01 to 0.2 mM.

The reaction rate is at least partially controlled by the rate at which oxygen can be dissolved into the aqueous medium. Oxygen can be added to the reaction as the oxygen in air, but it is preferred to use a relatively pure form of oxygen, and to use elevated pressures. Although no upper limit of oxygen pressure is known, oxygen pressures up to 50 atmospheres may be used, and an upper limit of 15 atmospheres is preferred. Sparging (bubbling) oxygen through the reaction mixture is necessary to maintain a high oxygen dissolution (and hence reaction) rate. Oxygen is sparged through the reaction mixture at a rate of 0.05 to 5 volumes of oxygen (measured at atmospheric pressure) per volume of reaction mixture per minute (vol/vol·min), and preferably between 0.2 and 2 vol/vol·min. Additionally, a convenient form of agitation is useful, such as stirring.

The reaction temperature is an important variable, in that it affects reaction rate and the stability of the enzymes. A reaction temperature of 0°C to 40°C may be used, but the preferred reaction temperature range is from 5°C to 15°C. Operating in the preferred temperature range maximizes recovered enzyme activity at the end of the reaction.

Upon completion of the reaction and removal of the microbial cell transformant catalyst by filtration or centrifugation, the amine buffer is most conveniently removed by use of an ion exchange resin. Suitable acidic cationic exchange resins include "AMBERLITE®" CG120 or "AMBERLITE®" IR120 (Rohm & Haas Co.), and "DOWEX®"50 (Dow Chemical Co.). The amine may then be recovered and subsequently recycled by treatment of the resin with strong base.

The product glyoxylic acid is useful in the preparation of vanillin and ethylvanillin, as well as being used in ion exchange resins and as an acid catalyst in the pharmaceutical industry (Ullmanns). It is usually sold as a 50% (weight percent) aqueous solution It is also to be understood that reference to glyoxylic acid in this application can also mean the glyoxylate anion, especially when the glyoxylic acid is present in a solution whose pH is greater than about 23.

### Media for Microbial Cell Transformants Cultured in Shaker Flask or Fermenter

The minimal media (MIN) used for culturing the microbial cell transformants consisted of fructose (1%, 1.0 g/L), threonine (100 mM, 11.9 g/L), ammonium tartrate (6.0 g/L), trace elements (1 mL/L, and salt solution (10 mL/L); the pH of this minimal media was adjusted to 6.5 with sodium hydroxide.

The rich (SYG) media used for culturing the microbial cell transformants consisted of yeast extract (0.5%, 5.0 g/L), ammonium nitrate (100 mM, 8.0 g/L), potassium phosphate (monobasic, 33 mM, 4.5 g/L), magnesium sulfate heptahydrate (2 mM, 0.5 g/L), trace elements (1.0 mL/L); after adjusting the pH to 5.5 and autoclaving, glucose was added to 2% (w/v).

### Glycolate Oxidase and Catalase Assays for Whole Cells

Microbial cell transformants were assayed for glycolate oxidase activity by accurately weighing ca. 5-10 mg of the wet cells (blotted on filter paper to remove excess moisture) into a 3-mL quartz cuvette containing a magnetic stirring bar and 2.0 mL of a solution which was 0.12 mM in 2,6-dichlorophenol-indophenol (DCIP) and 80 mM in TRIS buffer (pH 8.3). The cuvette was capped with a rubber septum and the solution deoxygenated by bubbling with nitrogen for 5 min. To the cuvette was then added by syringe 40 µL of 1.0 M glycolic acid/1.0 M TRIS (pH 8.3), and the mixture stirred while measuring the change in absorption with time at 605 nm (ε = 22,000).

Catalase activity was assayed by accurately weighing ca. 2-5 mg of the wet cells into a 3-mL quartz cuvette containing a magnetic stirring bar and 2.0 mL of a distilled water, then adding 1.0 mL of 50 mM hydrogen peroxide in 50 mM phosphate buffer (pH 7.0) and measuring the change in absorption with time at 240 nm (ε = 39.4). Glycolate oxidase and catalase activities of the *Aspergillus nidulans* wet cells cultured in different media ranged from 0.5 - 2.0 DCIP IU/gram for glycolate oxidase and 500 - 7000 IU/gram for catalase. Glycolate oxidase and catalase activities of the *E. coli* wet cells (unpermeabilized) cultured in different media ranged from 0.8 - 4.0 DCIP IU/gram wet cells for glycolate oxidase and 1000 - 2000 IU/gram wet cells for endogenous catalase. Glycolate oxidase and catalase activities of the *H. polymorpha* or *P. pastoris* wet cells (permeabilized) cultured in different media ranged from 20 - 120 DCIP IU/gram wet cells for glycolate oxidase and 30,000 - 200,000 IU/gram for endogenous catalase.

### HPLC Analysis for Glycolic, Glyoxylic, Oxalic, and Formic Acid

Samples for analysis were first filtered through a Millipore Ultrafree MC filter unit (10,000 mw cutoff). Analyses for glycolic acid, glyoxylic acid, oxalic acid and formic acid were performed by high performance liquid chromatography (HPLC) on a Bio-Rad Aminex HPX-87H column (300 x 7.8 mm) at 40°C, using as solvent an aqueous solution of H₂SO₄ (0.01 N) and 1-hydroxyethane-1,1-diphosphonic acid (0.1 mM) at 1.0 mL/minute. UV analysis was performed at 210 nm. The retention times for oxalic acid, glyoxylic acid, glycolic acid, formic acid, and propionic acid (internal standard) or isobutyric acid (internal standard) were 4.29, 6.09, 7.77, 8.79, 11.41, and 13.05 minutes, respectively.

### Example 1

Into a 20-mL pressure reaction bottle (Lab Glass #LG-3921-100) was placed 1.0 mL of a solution containing glycolic acid (0.750 M), ethylenediamine (0.866 M), propionic acid (0.075 M), and flavin mononucleotide (0.01 mM); the pH of this solution (ca. 9.2) was not adjusted. The solution was cooled to 5°C, then 200 mg of frozen *Aspergillus nidulans* T17 cells were added to the bottle. The bottle was fitted with a crown cap and septum (Lab Glass #LG-3922-100), and then pressurized to 70 psig and vented five times at 5°C with pure oxygen using a 22 gauge needle, then pressurized to 70 psig (483 kPa) with oxygen and the needle removed. The cap was checked for leaks by briefly submerging the tube in cold water and looking for gas bubbles, then wiped dry and placed upright in a test tube rack attached to the top of a rotary shaker. The contents of the bottle were shaken at 300 rpm for 6 hours at 5°C, then the bottle was vented, the cap removed, and the contents of the bottle transferred to a 1.5 mL microcentrifuge tube. The cells were briefly spun down, and a 100 µl aliquot of the supernatant analyzed by HPLC. The cell pellet was then assayed for recovered glycolate oxidase and catalase activity; recoveries of enzyme activities were based on the initial enzyme activities of the whole cells, and recoveries of greater than 100% are attributed to permeabilization of the cells over the course of the reaction.

| catalyst | time (h) | glyoxylate (%) | G.O. recovery (%) | catalase recovery (%) |
|---|---|---|---|---|
| ST17SYG | 6 | 45 | 134 | 119 |
| ST17SYG/OL | 6 | 65 | 309 | 316 |
| ST17SYG/OL2 | 6 | 51 | 847 | 254 |
| ST17SYG/OLHA | 6 | 24 | 219 | 180 |
| ST17SYCSL/OL | 6 | 53 | 102 | 60 |
| FT17SYG/OL | 6 | 47 | 164 | 79 |
| ST17MIN | 6 | 25 | 66 | 346 |
| ST18MIN | 6 | 14 | 13 | 390 |
| ST17SYG/OL | 23 | 100 | 0 | 597 |
| ST17SYCSL/OL | 23 | 100 | 0 | 64 |
| FT17SYG/OL | 23 | 100 | 144 | 157 |

OL = oleic acid

### Example 2

A 300-mL EZE-Seal stirred autoclave reactor (Autoclave Engineers) was charged with 75 mL of a solution containing glycolic acid (0.75 M), ethylenediamine (0.86 M, pH 9.2), propionic acid (0.075 M, HPLC internal standard), and flavin mononucleotide (0.01 mM), and the solution cooled to 15°C. To the reactor was then added 14 g of frozen (-80°C) *Aspergillus nidulans* ST17SYG/OL (25.2 IU glycolate oxidase and 20,400 IU catalase), and the cells were allowed to thaw at 15°C. The resulting mixture was stirred at 400 rpm and 15°C under 70 psig (483 kPa) of oxygen, while bubbling oxygen through the mixture at 20 mL/min. The reaction was monitored by taking a 100 µL aliquot of the reaction mixture at regular intervals, mixing the aliquot with 300 µL of 0.1 N sulfuric acid to quench the reaction, filtering the aliquot and analyzing by HPLC. After 7 hours, the yields of glyoxylic acid, oxalic acid, and formic acid were 79%, 0%, and 0%, respectively, with 2.7% recovery of glycolic acid. The final activities of glycolate oxidase and catalase were 55% and 80% of their initial values.

### Example 3

A 300-mL EZE-Seal stirred autoclave reactor (Autoclave Engineers) was charged with 100 mL of a solution containing glycolic acid (0.75 M), ethylenediamine (0.86 M, pH 92), propionic acid (0.075 M, HPLC internal standard), and flavin mononucleotide (0.01 mM), and the solution cooled to 5°C. To the reactor was then added 32 g of frozen (-80°C) *Aspergillus nidulan*s FT17SYG/OL (28.2 IU glycolate oxidase and 157,000 IU catalase), and the cells were allowed to thaw at 15°C. The resulting mixture was stirred at 400 rpm and 5°C under 70 psig (483 kPa) of oxygen, while bubbling oxygen through the mixture at 30 mL/min. The reaction was monitored by taking a 100 µL aliquot of the reaction mixture at regular intervals, mixing the aliquot with 300 µL of 0.1 N sulfuric acid to quench the reaction, filtering the aliquot and analyzing by HPLC. After 21 hours, the yields of glyoxylic acid, oxalic acid, and formic acid were 88.2%, 0%, and 0%, respectively, with 10.0% recovery of glycolic acid. The final activities of glycolate oxidase and catalase were 0% and 75% of their initial values.

### Example 4

A 300-mL EZE-Seal stirred autoclave reactor (Autoclave Engineers) was charged with 100 mL of a solution containing glycolic acid (0.75 M), ethylenediamine (0.86 M, pH 9.0), propionic acid (0.075 M, HPLC internal standard), and flavin mononucleotide (0.01 mM), and the solution cooled to 5°C. To the reactor was then added 26 g of frozen (-80°C) *Aspergillus nidulans* FT17SYG/OL (29.9 IU glycolate oxidase and 177,000 IU catalase), and the cells were allowed to thaw at 5°C. The resulting mixture was stirred at 400 rpm and 5°C under 70 psig (483 kPa) of oxygen, while bubbling oxygen through the mixture at 50 mL/min. The reaction was monitored by taking a 100 µL aliquot of the reaction mixture at regular intervals, mixing the aliquot with 300 µL of 0.1 N sulfuric acid to quench the reaction, filtering the aliquot and analyzing by HPLC. After 23 hours, the yields of glyoxylic acid, oxalic acid, and formic acid were 95%, 0%, and 0%, respectively, with complete conversion of glycolic acid. The final activities of glycolate oxidase and catalase were 12% and 76% of their initial values.

### Example 5

A 300-mL EZE-Seal stirred autoclave reactor (Autoclave Engineers) was charged with 100 mL of a solution containing glycolic acid (0.75 M), ethylenediamine (0.86 M, pH 9.0), propionic acid (0.075 M, HPLC internal standard), and flavin mononucleotide (0.01 mM), and the solution cooled to 5°C. To the reactor was then added 26 g of frozen (-80°C) *Aspergillus nidulans* FTI7SYG/OL (24 IU glycolate oxidase and 192,000 IU catalase), and the cells were allowed to thaw at 5°C. The resulting mixture was stirred at 400 rpm and 5°C under 120 psig of oxygen, while bubbling oxygen through the mixture at 50 mL/min. The reaction was monitored by taking a 100 µL aliquot of the reaction mixture at regular intervals, mixing the aliquot with 300 µL of 0.1 N sulfuric acid to quench the reaction, filtering the aliquot and analyzing by HPLC. After 115 hours, the yields of glyoxylic acid, oxalic acid, and formic acid were 98%, 0%, and 0%, respectively, with complete conversion of glycolic add. The final activities of glycolate oxidase and catalase were 100% and 62% of their initial values.

At the completion of the reaction, the reaction mixture was centrifuged at 5°C and the supernatant decanted. The resulting pellet of *Aspergillus nidulans* cells was resuspended in 100 mL of fresh reaction mixture at 5°C, and the reaction repeated under conditions identical to those described above. After 16 hours, the yields of glyoxylic acid, oxalic acid, and formic acid were 47%, 0%, and 0%, respectively, with a 54% recovery of glycolic acid. The recovered activities of glycolate oxidase and catalase at 16 hours were 91% and 100% of their initial values.

### Example 6

Into a 3 oz. Fischer-Porter glass aerosol reaction vessel was placed a magnetic stirring bar and 10 mL of an aqueous solution containing glycolic acid (0.750 M), ethylenediamine (0.863 M), isobutyric acid (0.100 M, HPLC internal standard), and flavin mononucleotide (0.01 mM) at pH 9.0, and the solution cooled to 5°C. To the vessel was then added 0.75 g of *Pichia pastoris* transformant strain GS115-MSP10 (31 IU glycolate oxidase and 38,100 IU catalase) which had been permeabilized by treatment with 0.1% "TRITON®" X-100/1 freeze-thaw, and the reaction vessel sealed and the reaction mixture was cooled to 5°C. The vessel was flushed with oxygen by pressurizing to 70 psig and venting to atmospheric pressure five times with stirring, then the vessel was pressurized to 70 psig of oxygen and the mixture stirred at 5°C. Aliquots (0.20 mL) were removed by syringe through a sampling port (without loss of pressure in the vessel) at regular intervals for analysis by HPLC to monitor the progress of the reaction. After 6 h, the HPLC yields of glyoxylate, formate, and oxalate were 98.2 %, 0 %, and 0 %, respectively, and no glycolate remained. The remaining permeabilized-cell glycolate oxidase and catalase activity were 85 % and 117 %, respectively, of their initial values.

The microbial cell catalyst was recovered from the reaction mixture described above by centrifugation. Without further treatment the cell pellet was mixed with 10 mL of fresh reaction mixture, and the reaction repeated. This catalyst recycle procedure was performed for ten consecutive batch reactions, and the reaction time, the recovery of catalase and glycolate oxidase activity (based on the initial activity of the permeabilized cells), and yields of glyoxylic, formic, oxalic, and glycolic acid are listed in the table below:

| run # | time (h) | catalase (%) | glycolate oxidase(%) | glyoxylic acid(%) | formic acid(%) | oxalic acid(%) | glycolic acid (%) |
|---|---|---|---|---|---|---|---|
| 1 | 6.0 | 117 | 85 | 98.2 | 0 | 0 | 0 |
| 2 | 4.0 | 78 | 78 | 99.6 | 0 | 0 | 0 |
| 3 | 4.0 | 68 | 68 | 97.1 | 0 | 1.3 | 0 |
| 4 | 4.0 | 72 | 73 | 99.5 | 0 | 0.5 | 0 |
| 5 | 3.0 | 77 | 74 | 99.2 | 0 | 0.5 | 0 |
| 6 | 4.5 | 71 | 71 | 99.0 | 0 | 0.5 | 0 |
| 7 | 5.5 | 70 | 74 | 98.0 | 0 | 2.0 | 0 |
| 8 | 5.0 | 72 | 61 | 99.5 | 0 | 0.5 | 0 |
| 9 | 5.5 | 60 | 48 | 98.6 | 0 | 1.4 | 0 |
| 10 | 5.5 | 56 | 42 | 99.1 | 0 | 0.2 | 0 |

### Example 7

A 300-mL EZE-Seal stirred autoclave reactor equipped with Dispersimax Impeller (Autoclave Engineers) was charged with 100 mL of a solution containing glycolic acid (0.750 M), ethylenediamine (0.863 M), isobutyric acid (0.100 M, HPLC internal standard), and flavin mononucleotide (0.01 mM) at pH 9.25, and the solution cooled to 5°C. To the reactor was then added 5.0 g of *Pichia pastoris* transformant strain GS115-MSP10 (423 IU glycolate oxidase and 869,000 IU catalase) which had been permeabilized by treatment with 0.1 % benzalkonium chloride (Sigma), and the reactor purged with oxygen. The mixture was then stirred at 1000 rpm, which bubbled oxygen through the mixture via the action of the turbine impeller, and at 5°C under 120 psig of oxygen. The reaction was monitored by taking a 0.40 mL aliquot of the reaction mixture at regular intervals, filtering the aliquot using a Millipore Ultrafree-MC 10,000 NMWL Filter Unit, and analyzing the filtrate by HPLC. After 1.0 h, the yields of glyoxylic acid, oxalic acid, and formic acid were 98.7 %, 13 %, and 0 %, respectively, with no remaining glycolic acid. The recovered activities of permeabilized-cell glycolate oxidase and catalase were 87 % and 84 % of their initial values, respectively.

The microbial cell catalyst was recovered from the reaction mixture described above by centrifugation Without further treatment the cell pellet was mixed with 100 mL of fresh reaction mixture, and the reaction repeated. This catalyst recycle procedure was performed for twenty consecutive batch reactions, and the reaction time, the recovery of catalase and glycolate oxidase activity (based on the initial activity of the permeabilized cells), and yields of glyoxylic, formic, oxalic, and glycolic acid are listed in the table below:

| run # | time (h) | catalase(%) | glycolate oxidase(%) | glyoxylic acid(%) | formic acid(%) | oxalic acid(%) | glycolic acid (%) |
|---|---|---|---|---|---|---|---|
| 1 | 1.0 | 84 | 87 | 98.7 | 0 | 1.3 | 0 |
| 2 | 1.0 | 88 | 104 | 98.7 | 0 | 1.3 | 0 |
| 3 | 1.0 | 85 | 107 | 98.8 | 0 | 1.2 | 0 |
| 4 | 1.0 | 79 | 126 | 98.7 | 0 | 1.3 | 0 |
| 5 | 1.0 | 69 | 104 | 98.8 | 0 | 1.2 | 0 |
| 6 | 1.0 | 79 | 109 | 98.9 | 0 | 1.1 | 0 |
| 7 | 1.0 | 71 | 110 | 99.3 | 0 | 0.7 | 0 |
| 8 | 1.0 | 64 | 113 | 99.2 | 0 | 0.8 | 0 |
| 9 | 1.0 | 61 | 106 | 99.4 | 0 | 0.6 | 0 |
| 10 | 1.0 | 61 | 101 | 99.1 | 0 | 0.9 | 0 |
| 11 | 1.0 | 72 | 104 | 99.5 | 0 | 0.5 | 0 |
| 12 | 1.0 | 68 | 99 | 99.4 | 0 | 0.6 | 0 |
| 13 | 1.5 | 70 | 101 | 99.3 | 0 | 0.7 | 0 |
| 14 | 1.5 | 59 | 96 | 99.6 | 0 | 0.4 | 0 |
| 15 | 1.5 | 58 | 86 | 99.6 | 0 | 0.4 | 0 |
| 16 | 1.75 | 58 | 83 | 99.6 | 0 | 0.4 | 0 |
| 17 | 2.0 | 56 | 77 | 97.2 | 0 | 2.8 | 0 |
| 18 | 2.0 | 37 | 91 | 99.7 | 0 | 0.3 | 0 |
| 19 | 2.5 | 50 | 73 | 99.7 | 0 | 0.3 | 0 |
| 20 | 3.5 | 46 | 72 | 99.9 | 0 | 0.1 | 0 |

### Example 8

A 300-mL EZE-Seal stirred autoclave reactor equipped with Dispersimax Impeller (Autoclave Engineers) was charged with 100 mL of a solution containing glycolic acid (0.750 M), ethylenediamine (0.863 M), isobutyric acid (0.100 M, HPLC internal standard), and flavin mononucleotide (0.01 mM) at pH 9.25, and the solution cooled to 5°C. To the reactor was then added 2.0 g of *Pichia pastoris* transformant strain GS115-MSP10 (276 IU glycolate oxidase and 494,000 IU catalase) which had been permeabilized by treatment with 0.1% Triton X-100/6 freeze-thaws, and the reactor purged with oxygen. The mixture was then stirred at 1000 rpm, which bubbled oxygen through the mixture via the action of the turbine impeller, and at 5°C under 120 psig of oxygen. The reaction was monitored by taking a 0.40 mL aliquot of the reaction mixture at regular intervals, filtering the aliquot using a Millipore Ultrafree-MC 10,000 NMWL Filter Unit, and analyzing the filtrate by HPLC. After 0.75 h, the yields of glyoxylic acid, oxalic acid, and formic acid were 99.1 %, 0.3 %, and 0 %, respectively, with 0.6 *%* glycolic acid remaining. The recovered activities of permeabilized-cell glycolate oxidase and catalase were 104 % and 105 % of their initial values, respectively.

The microbial cell catalyst was recovered from the reaction mixture described above by centrifugation. Without further treatment the cell pellet was mixed with 100 mL of fresh reaction mixture, and the reaction repeated. After 1.0 h, the yields of glyoxylic acid, oxalic acid, and formic acid were 99.7 %, 0.3 %, and 0 %, respectively, with no glycolic acid remaining. The recovered activities of permeabilized-cell glycolate oxidase and catalase were 101 % and 85 % of their initial values. This catalyst recycle procedure was performed for five consecutive batch reactions, and the reaction time, the recovery of catalase and glycolate oxidase activity (based on the initial activity of the permeabilized cells), and yields of glyoxylic, formic, oxalic, and glycolic acid are listed in the table below:

| run # | time (h) | catalase (%) | glycolate oxidase(%) | glyoxylic acid(%) | formic acid(%) | oxalic acid(%) | glycolic acid (%) |
|---|---|---|---|---|---|---|---|
| 1 | 0.75 | 105 | 104 | 99.1 | 0 | 0.3 | 0.6 |
| 2 | 1.0 | 85 | 101 | 99.7 | 0 | 0.3 | 0 |
| 3 | 1.5 | 82 | 97 | 99.6 | 0 | 0.4 | 0 |
| 4 | 1.5 | 67 | 96 | 99.8 | 0 | 0.2 | 0 |
| 5 | 2.0 | 92 | 93 | 99.7 | 0 | 0.3 | 0 |

### Example 9

A 300-mL EZE-Seal stirred autoclave reactor equipped with Dispersimax Impeller (Autoclave Engineers) was charged with 100 mL of a solution containing glycolic acid (1.500 M), ethylenediamine (1.575 M), isobutyric acid (0.300 M, HPLC internal standard), and flavin mononucleotide (0.01 mM) at pH 9.25, and the solution cooled to 5°C. To the reactor was then added 2.0 g of *Pichia pastoris* transformant strain GS115-MSP10 (114 IU glycolate oxidase and 148,000 IU catalase) which had been permeabilized by treatment with 0.1% Triton X-100/1 freeze-thaw, and the reactor purged with oxygen. The mixture was then stirred at 1000 rpm, which bubbled oxygen through the mixture via the action of the turbine impeller, and at 5°C under 120 psig of oxygen. The reaction was monitored by taking a 0.40 mL aliquot of the reaction mixture at regular intervals, filtering the aliquot using a Millipore Ultrafree-MC 10,000 NMWL Filter Unit, and analyzing the filtrate by HPLC. After 4.5 h, the yields of glyoxylic acid oxalic acid, and formic acid were 98.0 %, 0.4 %, and 0 %, respectively, with no glycolic acid remaining. The final activities of permeabilized-cell glycolate oxidase and catalase were 136 % and 113 % of their initial values, respectively.

### Example 10

Into a 3 oz. Fischer-Porter glass aerosol reaction vessel was placed a magnetic stirring bar and 10 mL of an aqueous solution containing glycolic acid (0.750 M), ethylenediamine (0.863 M), isobutyric acid (0.100 M, HPLC internal standard), and flavin mononucleotide (0.01 mM) at pH 9.0, and the solution cooled to 5°C. To the vessel was then added 0.47 g of *Hansenula polymorpha* transformant G01 (10.0 IU glycolate oxidase and 22,100 IU catalase) which had been permeabilized by treatment with 0.1% Triton X-100/1 freeze-thaw, and the reaction vessel sealed and the reaction mixture cooled to 5°C. The vessel was flushed with oxygen by pressurizing to 70 psig and venting to atmospheric pressure five times with stirring, then the vessel was pressurized to 70 psig of oxygen and the mixture stirred at 5°C. Aliquots (0.20 mL) were removed by syringe through a sampling port (without loss of pressure in the vessel) at regular intervals for analysis by HPLC to monitor the progress of the reaction. After 16 h, the HPLC yields of glyoxylate, formate, and oxalate were 97.1 *%,* 2.9 %, and 0 %, respectively, and no glycolate remained. The remaining permeabilized-cell glycolate oxidase and catalase activity were 107 % and 231 %, respectively, of their initial values.

### Example 11

A 300-mL EZE-Seal stirred autoclave reactor (Autoclave Engineers) was charged with 100 mL of a solution containing glycolic acid (0.750 M), ethylenediamine (0.863 M), isobutyric add (0.100 M, HPLC internal standard), and flavin mononucleotide (0.01 mM) at pH 93, and the solution cooled to 5°C. To the reactor was then added 11.9 g of *Hansenula polymorpha* transformant G01 (100 IU glycolate oxidase and 998,000 IU catalase) which had been permeabilized by treatment with 0.1% Triton® X-100/1 freeze-thaw, and the reactor purged with oxygen. The mixture was then stirred at 500 rpm and at 5°C under 120 psig of oxygen, and oxygen was bubbled through the mixture at 100 mL/min using a sparge tube located below the surface of the reaction mixture. The reaction was monitored by taking a 0.40 mL aliquot of the reaction mixture at regular intervals, filtering the aliquot using a Millipore Ultrafree-MC 10,000 NMWL Filter Unit, and analyzing the filtrate by HPLC. After 2.25 h, the yields of glyoxylic acid oxalic acid, and formic acid were 100 %, 0 %, and 0 %, respectively, with no glycolic acid remaining. The recovered activities of permeabilized-cell glycolate oxidase and catalase were 158 % and 82% of their initial values, respectively.

### Example 12

The reaction in Example 6 was repeated using 15.0 g of *Hansenula polymorpha* transformant G01 (109 IU glycolate oxidase an 530,000 IU catalase) which had been permeabilized by treatment with 0.1% Triton® X-100/1 freeze-thaw. The mixture was stirred at 500 rpm and at 5°C under 120 psig of oxygen, and oxygen was bubbled through the mixture at 50 mL/min using a sparge tube located below the surface of the reaction mixture. After 3.75 h, the yields of glyoxylic acid, oxalic acid, and formic acid were 100 %, 0 %, and 0 %, respectively, with no glycolic acid remaining. The recovered activities of permeabilized-cell glycolate oxidase and catalase were 85 % and 166 % of their initial values, respectively.

### Example 13

The reaction in Example 6 was repeated using 15.0 g of *Hansenula polymorpha* transformant G01 (51 IU glycolate oxidase and 730,000 IU catalase) which had been permeabilized by treatment with 0.1% Triton X-100/1 freeze-thaw. The mixture was stirred at 1250 rpm, which bubbled oxygen through the mixture via the action of the Dispersimax turbine impeller, and at 5°C under 120 psig of oxygen. After 4.0 h, the yields of glyoxylic acid, oxalic acid, and formic acid were 97.5 %, 0 %, and 0 %, respectively, with 0.6 % glycolic acid remaining. The recovered activities of permeabilized-cell glycolate oxidase and catalase were 132 % and 129 % of their initial values, respectively.

### Example 14

A 300-mL EZE-Seal stirred autoclave reactor equipped with Dispersimax Impeller (Autoclave Engineers) was charged with 100 mL of a solution containing glycolic acid (0.750 M), ethylenediamine (0.863 M), isobutyric acid (0.100 M, HPLC internal standard), and flavin mononucleotide (0.01 mM) at pH 9.3, and the solution cooled to 5°C. To the reactor was then added 15.0 g of *Hansenula polymorpha* transformant G01 (262 IU glycolate oxidase and 1.135 x 10⁶ IU catalase) which had been permeabilized by treatment with 0.1% Triton X-100/1 freeze-thaw, and the reactor purged with oxygen. The mixture was then stirred at 1000 rpm, which bubbled oxygen through the mixture via the action of the turbine impeller, and at 5°C under 250 psig of oxygen. The reaction was monitored by taking a 0.40 mL aliquot of the reaction mixture at regular intervals, filtering the aliquot using a Millipore Ultrafree-MC 10,000 NMWL Filter Unit, and analyzing the filtrate by HPLC. After 1.0 h, the yields of glyoxylic acid, oxalic acid, and formic acid were 96.9 %, 0.3 %, and 0 %, respectively, with no remaining glycolic acid. The recovered activities of permeabilized-cell glycolate oxidase and catalase were 98 % and 124 % of their initial values, respectively.

The microbial cell catalyst was recovered from the reaction mixture described above by centrifugation. Without further treament the cell pellet was mixed with 100 mL of fresh reaction mixture, and the reaction repeated. This catalyst recycle procedure was performed for eight consecutive batch reactions, and the reaction time, the recovery of catalase and glycolate oxidase activity (based on the initial activity of the permeabilized cells), and yields of glyoxylic, formic, oxalic, and glycolic acid are listed in the table below:

| run # | time (h) | catalase (%) | glycolate oxidase(%) | glyoxylic acid(%) | formic acid(%) | oxalic acid(%) | glycolic acid (%) |
|---|---|---|---|---|---|---|---|
| 1 | 1.0 | 124 | 98 | 96.9 | 0 | 0.3 | 0 |
| 2 | 1.5 | 145 | 84 | 99.6 | 0 | 0.4 | 0 |
| 3 | 2.0 | 162 | 77 | 97.4 | 0 | 0.3 | 0 |
| 4 | 2.0 | 117 | 57 | 94.6 | 0 | 1.0 | 0 |
| 5 | 2.5 | 128 | 44 | 97.7 | 0 | 0.7 | 0 |
| 6 | 3.0 | 133 | 40 | 96.6 | 0 | 0.1 | 0 |
| 7 | 5.0 | 111 | 23 | 99.1 | 0 | 0.2 | 0 |
| 8 | 16.5 | 116 | 19 | 95.2 | 0 | 0.3 | 0 |

### Example 15

The reaction in Example 9 was repeated except that FMN was not added to the reaction mixture. The catalyst was 5.0 g of *Hansenula polymorpha* transformant G01 (880 IU glycolate oxidase and 453,000 IU catalase) which had been permeabilized by treatment with 0.1% Triton® X-100/1 freeze-thaw. The catalyst recycle procedure was performed for twenty consecutive batch reactions with no added FMN, and the reaction time, the recovery of catalase and glycolate oxidase activity (based on the initial activity of the permeabilized cells), and yields of glyoxylic, formic, oxalic, and glycolic acid are listed in the table below:

| run # | time (h) | catalase(%) | glycolate oxidase(%) | glyoxylic acid(%) | formic acid(%) | oxalic acid(%) | glycolic acid (%) |
|---|---|---|---|---|---|---|---|
| 1 | 1.0 | 100 | 100 | 96.9 | 0.1 | 1.1 | 1.2 |
| 2 | 1.0 | 88 | 109 | 98.4 | 0.1 | 1.2 | 1.4 |
| 3 | 1.0 | 102 | 110 | 98.2 | 0.1 | 1.0 | 0.9 |
| 4 | 1.0 | 103 | 107 | 98.0 | 0.1 | 1.0 | 0.9 |
| 5 | 1.0 | 86 | 90 | 97.8 | 0.2 | 1.1 | 1.1 |
| 6 | 1.0 | 85 | 95 | 98.4 | 0.1 | 0.9 | 1.1 |
| 7 | 1.0 | 89 | 116 | 97.9 | 0.1 | 0.9 | 1.1 |
| 8 | 1.3 | 89 | 116 | 99.1 | 0.1 | 1.1 | 1.0 |
| 9 | 1.0 | 87 | 103 | 98.0 | 0.1 | 1.0 | 1.0 |
| 10 | 1.0 | 106 | 116 | 98.3 | 0.1 | 0.8 | 0.8 |
| 11 | 1.0 | 85 | 104 | 97.9 | 0.1 | 0.8 | 0.9 |
| 12 | 1.5 | 99 | 101 | 96.6 | 0.1 | 0.8 | 1.0 |
| 13 | 1.5 | 98 | 105 | 98.1 | 0.1 | 0.7 | 1.0 |
| 14 | 1.0 | 78 | 85 | 98.5 | 0.1 | 0.6 | 1.8 |
| 15 | 1.0 | 88 | 82 | 98.3 | 0.2 | 0.5 | 1.1 |
| 16 | 1.0 | 90 | 82 | 99.6 | 0.1 | 0.5 | 0.8 |
| 17 | 1.0 | 59 | 56 | 98.8 | 0 | 0.5 | 1.0 |
| 18 | 1.0 | 48 | 60 | 97.7 | 0.6 | 0.4 | 1.5 |
| 19 | 1.0 | 54 | 63 | 98.6 | 0.1 | 0.6 | 1.7 |
| 20 | 1.5 | 86 | 61 | 98.0 | 0.1 | 0.7 | 1.3 |

### Example 16

A 300-mL EZE-Seal stirred autoclave reactor equipped with Dispersimax Impeller (Autoclave Engineers) was charged with 100 mL of a solution containing glycolic acid (0.750 M), ethylenediamine (0.863 M), isobutyric acid (0.100 M, HPLC internal standard), and flavin mononucleotide (0.01 mM) at pH 9.2, and the solution cooled to 5°C. To the reactor was then added 30 g of *E. coli* transformant d01 (72 IU glycolate oxidase and 29,600 IU catalase), and the mixture stirred at 1000 rpm, which bubbled oxygen through the mixture via the action of the turbine impeller, and at 5°C under 120 psig of oxygen. The reaction was monitored by taking a 0.40 mL aliquot of the reaction mixture at regular intervals, filtering the aliquot using a Millipore Ultrafree-MC 10,000 NMWL Filter Unit, and analyzing the filtrate by HPLC. After 23 h, the yields of glyoxylic acid, oxalic acid, and formic acid were 74.4 %, 1.1 %, and 5.6 %, respectively, with 6.3 % glycolic acid remaining. The recovered activities of microbial glycolate oxidase and catalase were 30 % and 199 % of their initial values, respectively.

## Claims

1. A process for the production of glyoxylic acid comprising the contacting, in aqueous solution, glycolic acid with oxygen in the presence of an effective amount of whole microbial cells which as a result of genetic engineering expresses the enzyme glycolate oxidase, (S)-2-hydroxy-acid oxidase, achieving an effective concentration range of 0.01 to about 100 IU/mL of glycolate oxidase for sufficient time to catalytically convert glycolic acid to glyoxylic acid and thereafter recovering glyoxylic acid.

2. A process of claim 1 wherein said whole microbial cells co-express the enzyme catalase achieving an effective concentration range of 50 to 100,000 IU/mL of catalase.

3. A process of claim 2 wherein said contacting of glycolic acid with oxygen is at a pH of about 7 to 10 in the presence of an amine capable of forming an adduct with glyoxylic acid wherein the initial molar ratio of amine to glycolic acid is from 1.0 to 3.0, and said glycolic acid is initially present at a concentration of about 200 mM to 2500 mM.

4. A Process of claim 2 wherein the selectivity of glyoxylic acid production is at least 99%.

5. A Process of claim 4 wherein the concentration of glycolic oxidase is from 0.1 to 10 IU/mL.

6. A Process of claim 5 wherein the concentration of catalase is from 350 to 14,000 IU/mL.

7. A Process of claim 6 wherein the reaction is carried out at 0° to 40°C.

8. A Process of claim 7 wherein the reaction is carried out at an oxygen pressure of from atmospheric to 50,658 bar (50 atmospheres).

9. A Process of claim 8 wherein the temperature is from 5° to 15°C

10. A Process of claim 9 wherein the reaction is carried out at a pressure of from 1,013 bar to 15,197 bar (1 to 15 atmospheres).

11. A Process of claim 3 wherein the amine is ethylenediamine.

12. A Process of claim 3 wherein the amine is tris(hydroxymethyl)aminomethane.

13. A Process of claim 3 wherein the amine is piperazine.

14. A Process of claim 3 wherein the amine is glycylglycine.

15. A process of claim 1 wherein the whole microbial cells are a genetically-engineered transformant of *Aspergillus nidulans.*

16. A process of claim 1 wherein the whole microbial cells are a genetically-engineered transformant of *Pichia pastoris.*

17. A process of claim 1 wherein the whole microbial cells are a genetically-engineered transformant of *Hansenula polymorpha*.

18. A process of claim 1 wherein the whole microbial cells are a genetically-engineered transformant of *Escherichia coli*.

## Patentansprüche

1. Verfahren zur Herstellung von Glyoxalsäure, umfassend das Kontaktieren in wäßriger Lösung von Glykolsäure mit Sauerstoff in Gegenwart einer effektiven Menge ganzer mikrobieller Zellen, welche als Ergebnis gentechnischer Veränderung das Enzym Glykolat-Oxidase, (S)-2-Hydroxysäure-Oxidase, exprimieren, wobei sie einen effektiven Konzentrationsbereich von 0,01 bis etwa 100 IE/ml an Glykolat-Oxidase erreichen, für eine ausreichende Zeit, um Glykolsäure katalytisch in Glyoxalsäure zu überführen, und die anschließende Gewinnung von Glyoxalsäure.

2. Verfahren nach Anspruch 1, worin die ganzen mikrobiellen Zellen das Enzym Katalase koexprimieren, wobei sie einen effektiven Konzentrationsbereich von 50 bis 100 000 IE/ml an Katalase erreichen.

3. Verfahren nach Anspruch 2, worin das Kontaktieren von Glykolsäure mit Sauerstoff bei einem pH von etwa 7 bis 10 in Gegenwart eines Amins geschieht, welches imstande ist, mit Glyoxalsäure ein Addukt zu bilden, wobei das anfängliche Molverhältnis von Amin zu Glykolsäure 1,0 bis 3,0 beträgt und die Glykolsäure anfangs in einer Konzentration von etwa 200 mM bis 2500 mM vorhanden ist.

4. Verfahren nach Anspruch 2, worin die Selektivität der Glyoxalsäureproduktion mindestens 99% beträgt.

5. Verfahren nach Anspruch 4, worin die Konzentration an Glykolat-Oxidase 0,1 bis 10 IE/ml beträgt.

6. Verfahren nach Anspruch 5, worin die Konzentration an Katalase 350 bis 14 000 IE/ml beträgt.

7. Verfahren nach Anspruch 6, worin die Umsetzung bei 0° bis 40°C durchgeführt wird.

8. Verfahren nach Anspruch 7, worin die Umsetzung bei einem Sauerstoffdruck von Atmosphärendruck bis 50,658 bar (50 Atmosphären) durchgeführt wird.

9. Verfahren nach Anspruch 8, worin die Temperatur 5° bis 15°C beträgt.

10. Verfahren nach Anspruch 9, worin die Umsetzung bei einem Druck von 1,013 bar bis 15,197 bar (1 bis 15 Atmosphären) durchgeführt wird.

11. Verfahren nach Anspruch 3, worin das Amin Ethylendiamin ist.

12. Verfahren nach Anspruch 3, worin das Amin Tris(hydroxymethyl)aminomethan ist.

13. Verfahren nach Anspruch 3, worin das Amin Piperazin ist.

14. Verfahren nach Anspruch 3, worin das Amin Glycylglycin ist.

15. Verfahren nach Anspruch 1, worin die ganzen mikrobiellen Zellen eine gentechnisch veränderte Transformante von *Aspergillus nidulans* sind.

16. Verfahren nach Anspruch 1, worin die ganzen mikrobiellen Zellen eine gentechnisch veränderte Transformante von *Pichia pastoris* sind.

17. Verfahren nach Anspruch 1, worin die ganzen mikrobiellen Zellen eine gentechnisch veränderte Transformante von *Hansenula polymorpha* sind.

18. Verfahren nach Anspruch 1, worin die ganzen mikrobiellen Zellen eine gentechnisch veränderte Transformante von *Escherichia coli* sind.

## Revendications

1. Un procédé pour la production d'acide glyoxylique consistant à mettre en contact, dans une solution aqueuse, de l'acide glycolique avec de l'oxygène en présence d'une quantité efficace de cellules microbiennes entières qui, suite à une transformation par génie génétique, expriment l'enzyme glycolate-oxydase, (S)-2-hydroxyacide-oxydase, en atteignant un intervalle de concentration efficace de 0,01 à environ 100 UI/ml de glycolate-oxydase pendant un temps suffisant pour convertir catalytiquement l'acide glycolique en acide glyoxylique, puis à recueillir l'acide glyoxylique.

2. Un procédé de la revendication 1, dans lequel lesdites cellules microbiennes entières expriment conjointement l'enzyme catalase en atteignant un intervalle de concentration efficace de 50 à 100 000 UI/ml de catalase.

3. Un procédé de la revendication 2, dans lequel ladite mise en contact de l'acide glycolique avec l'oxygène s'effectue à un pH d'environ 7 à 10 en présence d'une amine capable de former un produit d'addition avec l'acide glyoxylique, le rapport molaire initial de l'amine à l'acide glycolique étant de 1,0 à 3,0, et ledit acide glycolique étant initialement présent à une concentration environ 200mM à 2500mM.

4. Un procédé de la revendication 2, dans lequel la sélectivité de production d'acide glyoxylique est d'au moins 99 %.

5. Un procédé de la revendication 4, dans lequel la concentration de glycolate-oxydase est de 0,1 à 10 UI/ml.

6. Un procédé de la revendication 5, dans lequel la concentration de catalase est de 350 à 14 000 UI/ml.

7. Un procédé de la revendication 6, dans lequel la réaction est conduite entre 0° et 40°C.

8. Un procédé de la revendication 7, dans lequel la réaction et conduite à une pression d'oxygène comprise entre la pression atmosphérique et 50,658 bars.

9. Un procédé de la revendication 8, dans lequel la température est de 5° à 15°C.

10. Un procédé de la revendication 9, dans lequel la réaction est conduite à une pression de 1,013 bar à 15,197 bars.

11. Un procédé de la revendication 3, dans lequel l'amine est l'éthylènediamine.

12. Un procédé de la revendication 3, dans lequel l'amine est le tris(hydroxyméthyl)aminométhane.

13. Un procédé de la revendication 3, dans lequel l'amine est la pipérazine.

14. Un procédé de la revendication 3, dans lequel l'amine est la glycylglycine.

15. Un procédé de la revendication 1, dans lequel les cellules microbiennes entières sont un transformant de *Aspergillus nidulans* obtenu par génie génétique.

16. Un procédé de la revendication 1, dans lequel les cellules microbiennes entières sont un transformant de *Pichia pastoris* obtenu par génie génétique.

17. Un procédé de la revendication 1, dans lequel les cellules microbiennes entières sont un transformant de *Hansenula polymorpha* obtenu par génie génétique.

18. Un procédé de la revendication 1, dans lequel les cellules microbiennes entières sont un transformant de *Escherichia coli* obtenu par génie génétique.
